# EUROPEAN PATENT SPECIFICATION

(11) **EP 2 224 881 B1**
(45) Date of publication and mention of the grant of the patent: **23.04.2014**
(21) Application number: 08806757.4
(22) Date of filing: 16.10.2008
(51) Int. Cl.: A61F 2/14, A61F 9/007, A61F 9/01

(54) **OPHTHALMIC SURGICAL DEVICE FOR ENDOTHELIAL KERATOPLASTY FOR DESCEMET'S STRIPPING AUTOMATED ENDOTHELIAL KERATOPLASTY (DSAEK) SURGERY**
OPHTHALMISCHE CHIRURGISCHE VORRICHTUNG FÜR DIE ENDOTHELIAL-KERATOPLASTIE FÜR DIE POSTERIORE LAMELLÄRE KERATOPLASTIK MITTELS DESCEMETSTRIPPING UND MIKROKERATOM (DSAEK)
DISPOSITIF CHIRURGICAL OPHTALMIQUE POUR KÉRATOPLASTIE ENDOTHÉLIALE POUR INTERVENTION CHIRURGICALE DSAEK (DESCEMET'S STRIPPING AUTOMATED ENDOTHELIAL KERATOPLASTY)

(30) Priority: 16.10.2007 US 980292 P
(43) Date of publication of application: 08.09.2010
(73) Proprietor: Tan, Donald, Singapore 267054 (SG); Mehta, Jod, Singapore 138686 (SG); Yong, Ming Por, Singapore 596289 (SG); Weston, Philip Douglas, Grantley, North Yorkshire HG4 3PJ (GB)
(72) Inventor: Tan, Donald, Singapore 267054 (SG); Mehta, Jod, Singapore 138686 (SG); Yong, Ming Por, Singapore 596289 (SG); Weston, Philip Douglas, Grantley, North Yorkshire HG4 3PJ (GB)
(74) Representative: Vaughan, Christopher Tammo
(86) International application number: PCT/GB2008/050943
(87) International publication number: WO 2009/050511

(56) References cited:
- WO-A-2006/029316
- WO-A-2008/141309
- US-A- 5 312 413
- US-A1- 2007 244 559
- US-B2- 6 858 033

## Description

### BACKGROUND

A paradigm shift in the approach to corneal transplantation is occurring, with new forms of anterior and posterior lamellar keratoplasty now enabling targeted replacement of only diseased layers of the cornea. These forms of lamellar corneal surgery are gradually replacing conventional full thickness penetrating keratoplasty (Tan DT, Mehta JS: "Future Directions in Lamellar Corneal Transplantation"; Cornea; in press).

Descemet's stripping automated endothelial keratoplasty (DSAEK) is a form of small incision and essentially sutureless surgery which represents the latest innovation in a series of posterior lamellar keratoplasty procedures that are now synonymous with the term "endothelial keratoplasty". The DSAEK procedure involves stripping of diseased Descemet's membrane and endothelial cells through a small corneal incision, and replacement with a posterior lamellar donor corneal lenticule prepared with the use of the Automated Lamellar Therapeutic Keratoplasty (ALTK) unit (Price MO, Price FW Jr.: "Descemet's stripping with endothelial keratoplasty: comparative outcomes with microkeratome-dissected and manually dissected donor tissue"; Ophthalmology; 2006 Nov; 113(11):1936-42).

With the adoption of any new surgical technique there is an inevitable learning curve for the surgeon and an accompanying evolution in techniques (see, for example: .Price FW, Price MO: "Descemet's stripping with endothelial keratoplasty in 200 eyes: Early challenges and techniques to enhance donor adherence"; J Cataract Refract Surg. 2006; 32(3):411-8; Melles GR, Lander F, Beekhuis WH, Remeijer L, Binder PS: "Posterior lamellar keratoplasty for a case of pseudophakic bullous keratopathy"; Am J Ophthalmol. 1999 Mar; 127(3):340-1; Melles GR, Lander F, Nieuwendaal C: "Sutureless, posterior lamellar keratoplasty: a case report of a modified technique"; Cornea; 2002 Apr; 21 (3):325-7; Melles GR, Wijdh RHJ, Nieuwendaal CP: "A technique to excise the Descemet membrane from a recipient cornea (descemetorhexis)"; Cornea; 2004 Apr; 23(3):286-8; Terry MA, Ousley PJ: "Replacing the endothelium without corneal surface incisions or sutures: the first United States clinical series using the deep lamellar endothelial keratoplasty procedure"; Ophthalmology; 2003 Apr; 110:755-64; discussion 764).

WO 2006/029316 discloses an ocular device holder for holding an ocular device (such as an implant) and to assist its placement into the anterior chamber of an eye.

US 5,312,413 discloses a tool for ophthalmic surgery including a shiftable guard or shield to cover a sharp protruding end of the tool.

US 6,858,033 discloses an insertion system for inserting an intraocular lens by way of an insertion tube and a pusher.

One of the most challenging aspects of this procedure is the insertion of the donor posterior lenticule into the anterior chamber (AC) through a small incision, without inducing significant endothelial damage. The current widely performed technique requires insertion of the donor lenticule through a small 5mm corneal or scleral incision by folding the lenticule and gripping the folded tissue with non-compressing forceps i.e. 'taco insertion'. This traumatic handling of the donor has been criticized because of its propensity for damaging endothelial cells, with primary graft failure rates due to intraoperative endothelial cell loss and damage ranging from 6% to 45% in the current literature with this folding technique (Mearza AA, Qureshi MA, Rostron CK: "Experience and 12-month results of Descemet-stripping endothelial keratoplasty (DSEK) with a small-incision technique"; Cornea 2007 Apr; 26(3):279-283). Damage to endothelial cells may occur as a consequence of mechanical folding of the donor, compression with holding forceps, and may also occur during intraocular manipulations to unfold the donor within the AC without the presence of an ophthalmic visco-surgical device (OVD). More recently, laboratory models of DSAEK have shown that folding of the donor lenticule for insertion into the AC and intraocular manipulation to unfold the donor is the stage most associated with significant endothelial cell loss (Lee WB, Sy HM, Holley GP, Edelhauser HF: "Descemet's Stripping Automated Endothelial Keratoplasty (DSAEK): Intra-Operative Effects on the Donor Corneal Endothelium"; IOVS supplement; 2007; abstract 1131). The endothelial damage is worse in the presence of associated anterior chamber shallowing.

Our own extensive in-vitro work has confirmed that significant endothelial damage occurs with the conventional folding technique, despite the use of commercially available 'non-compression' forceps (Goosey forceps, model no. 19090, Moria, Antony, France). Damage primarily occurring as a consequence of direct contact of folded endothelial surfaces where the folding forceps are applied, as well as along the folding crease (Mehta JS, Por YM, Beuerman RW, Tan DT: "Glide Insertion Technique of Donor Cornea Lenticule during Descemet's Stripping Automated Endothelial Keratoplasty"; J Cat Refract Surg; in press). Our recent studies show that the mean endothelial cell loss is 39% with this technique, which is now described:

A 1 mm paracentesis is first made in the peripheral cornea opposite a 5mm temporal scleral tunnel wound (for insertion of intraocular forceps). A standard, commercially available anterior chamber intraocular lens (IOL) Sheet's glide is trimmed to 4mm in width along approximately half to 2/3 of its length. Using Kelman Macpherson forceps, the glide is inserted into the AC through the scleral tunnel, with the right hand, whilst a balanced saline solution (BSS) infusion is maintained on. The donor (both the anterior and posterior lamellae) is transferred to a Paton's spatula. A dispersive OVD is liberally applied over the endothelial surface particularly the peripheral circumference of the donor. Carefully gripping the posterior donor lamellar with Kawai intraocular capsulorhexis forceps (Asico) on the stroma side, the anterior cap is slid away from the spatula, ensuring that the posterior donor lamella stays on the spatula. OVD is placed on the anterior surface of the glide, and the Paton spatula with the posterior lenticule is carefully everted, corneal endothelial surface down, onto the OVD-covered portion of the glide. Holding the glide with the right hand with Kelman Macpherson forceps at its most posterior part, the left hand, passes the Kawai forceps through the paracentesis, across the AC and over the sheets glide, and is passed out through the scleral incision. The Kawai forceps is rotated, so that the forceps teeth are now obliquely or vertically aligned, and can be used to grasp the leading edge of the donor lamella, on the upper stromal surface. Once the forceps grasped the donor edge, the donor is rapidly pulled through the scleral incision in one steady, smooth motion until the donor is fully in the AC. At the same time, the glide was retracted out of the eye.

We have performed this technique in 24 cases of DSAEK surgery, with only one primary graft failure occurring (4.2%). This contrasts with our previous 20 cases using the folding technique which had primary graft failure rate of 25% (5 eyes). Our scanning electron microscope (SEM) studies confirm that significant reduction in endothelial loss occurs with this technique, with a mean cell loss of 9%, mostly occurring at the peripheral rim, which may be due to contact of the donor edges with the plastic sheets glide, despite the use of OVD, and some damage must still occur when the donor is dragged through the lips of the wound, as the donor endothelial surface is still potentially in contact with the inferior lip of the scleral wound. We have not encountered any cases of donor dislocation with this technique, although we have now seen one case of partial Descemet's detachment. Our only primary graft failure occurred during our first case using this technique and can be attributed to the use of an excessively thick donor lenticule (400µm) which resulted in Descemet's detachment.

### BRIEF SUMMARY OF THE DISCLOSURE

There is provided an assembly for resiliently deforming a corneal implant comprising:
a housing member having an internal curved surface, said housing member arranged to receive and hold said implant in a curved orientation, such that peripheral edges of said implant are directed away from said housing member;
a platform in sliding engagement with said housing member, said platform having a profiled face arranged to contact said implant on sliding said housing member into contact with said profiled face; wherein
said profiled face shaped to direct said peripheral edges towards said housing member on contact through said sliding engagement.

In a preferred embodiment, the pre-determined shape may have a large radius of curvature, so as to prevent kinking or other subsequent damage to the implant.

In a further preferred embodiment, the pre-determined shape may be any one of: a double coil, a cardioid, reverse curve, reverse double curve or corrugated.

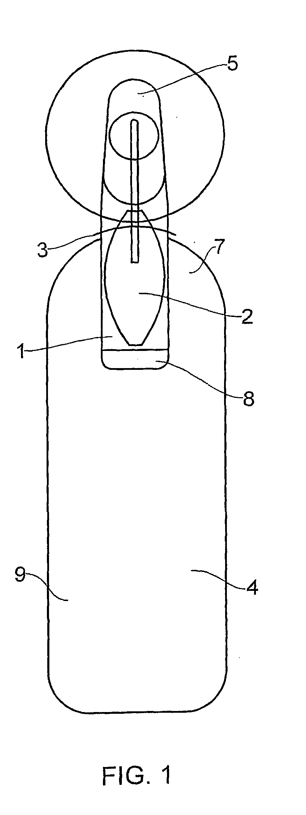

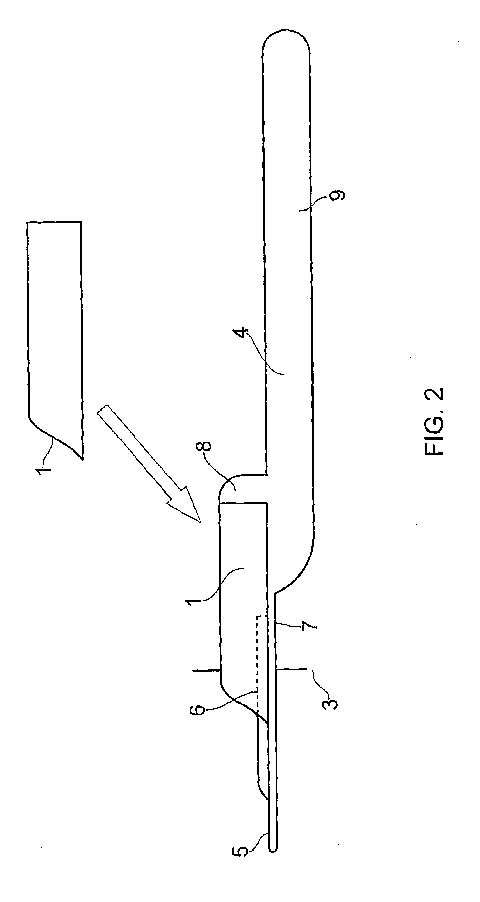

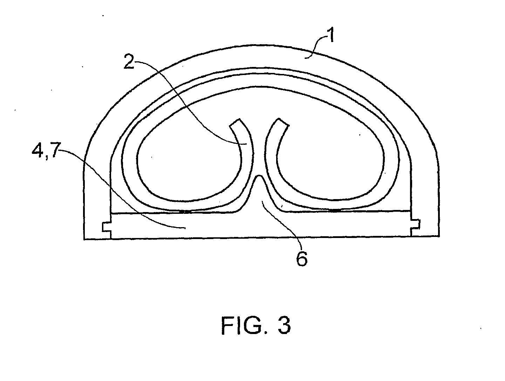

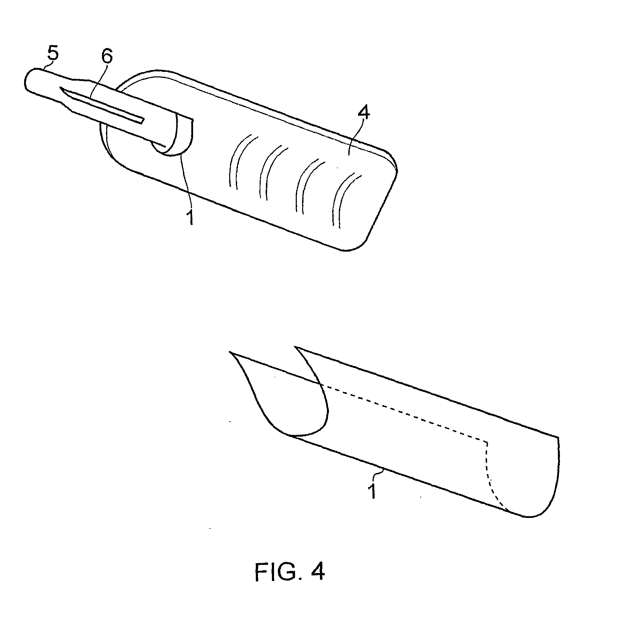

In a preferred embodiment, the housing member may be an elongate curved member such as that shown as the donor chamber of Figures 1 to 4. Alternatively, the member may have a range of external shapes, whilst maintaining a curved inner shape for contact with said implant.

In a preferred embodiment, the platform may be an elongate member such as that shown as the glide platform of Figures 1, 2 and 4.

In a preferred embodiment, the biasing member may be forceps arranged to engage and pull the implant into place.

Alternatively, the biasing means may include an element, such as a linearly directed projection, for pushing the implant into place. The element may take the form of a piston which fits in the deformation chamber or at least the housing, and which can be operated so as to push or eject the implant from the deformation chamber and into the eye of a recipient of the implant through the corneal incision. This embodiment is constructed in similar manner to a syringe. The element may alternatively take the form of a plate located at an end of a push-rod, the push-rod being connected to the plate at an edge thereof. This embodiment may be constructed in similar manner to a croupier's rake or croupier's tool. In both of these embodiments, the element may be held stationary relative to the recipient of the implant while the deformation chamber or at least the housing is moved backwards away from the incision, or the deformation chamber or at least the housing may be held stationary while the element is moved towards the incision.

In a further embodiment, the biasing means may engage a surface of the implant, through friction adhesion or other such means, and draw the implant from the assembly into place.

Embodiments of the present invention provide a disposable, plastic (or other appropriate surgical grade material, such as stainless steel) glide inserter which aims to simplify donor lenticule insertion in endothelial keratoplasty, and to reduce endothelial cell loss to below 5%.

Utilising the glide concept enables insertion of the donor lenticule into the AC with no significant shallowing or loss of the AC, despite no OVD being present in the AC. An AC maintainer is preferably used to provide a constant flow of BSS to maintain the AC throughout the insertion procedure.

The glide prevents iris prolapse and also protects the endothelial surface of the donor from contact with iris tissue, and an AC IOL, if present. Insertion of the thin, flat glide results in minimal loss of BSS from the wound, and provides a stable, deep chamber at all times.

The novel device of certain embodiments, which can be an intrinsic part of the glide, is a transparent, closed chamber which is inserted into the wound with a complete seal, thus again providing a deep chamber with minimal BSS outflow.

Transparent plastic used in the device allows for clear visualization of the donor at all times.

In preferred embodiments, a fully closed chamber environment (consisting of the AC and the internal cavity of the glide ensures full stability and minimal BSS flow, enabling the intraocular forceps to pull the donor into the AC in a controlled fashion with no risk of chamber collapse.

The internal diameter of the glide chamber may be calculated to allow for a donor lenticule of 9.5mm in diameter with a thickness of up to 250µm and in some cases up to 400µm or greater to be coiled up and inserted with no endothelial contact with either endothelial surfaces, wound edges, or intraocular surfaces.

The donor can be double coiled in a novel fashion to ensure no contact between endothelial surfaces at any time during the insertion procedure.

Several sizes (small, medium, large) of the glide device may be provided to allow surgeon selection depending on wound size, donor diameter and donor thickness

Embodiments of the present invention may be provided prepacked in a sterile package with a plastic (or other appropriate material, such as metal) preparation base which also functions as a base for coiling the donor.

The whole package may consist of two clear plastic components: a) Donor Chamber, b) Glide Platform, which come together to form a fully assembled Thumb Glide, and c) Preparation Base, which is useful for loading of the cornea within the Donor Chamber and for assembling the Thumb Glide of embodiments of the invention. Special glide forceps are a separate but also useful component of the procedure, but are not described in detail here.

The donor chamber or deformation chamber may be adapted so that it is sealable, for example by way of a screw cap or plug or stopper at one or both ends. In this way, a corneal implant can be prepared at a remote location, for example an eye bank, and stored the predetermined deformed shape in an appropriate nutrient solution or saline solution in a donor chamber or deformation chamber that is subsequently sealed. The sealed chamber can then be shipped to a surgeon in ready-to-use form. The surgeon then need only remove the seal(s) from the chamber before inserting the implant by way of the present invention.

### BRIEF DESCRIPTION OF THE DRAWINGS

For a better understanding of the present invention and to show how it may be carried into effect, reference shall now be made by way of example to the accompanying drawings, in which:
FIGURE 1 is a plan view of a first embodiment;
FIGURE 2 is a side elevation of the first embodiment;
FIGURE 3 is a cross-sectional view through the first embodiment;
FIGURE 4 is a perspective view of the first embodiment;
FIGURE 5 is a side elevation showing the first embodiment mounted on a preparation base for loading of a donor lenticule;
FIGURE 6 is a plan view of the preparation base of Figure 5;
FIGURE 7 shows a variation of the donor chamber with a croupier's rake biasing means;
FIGURE 8 shows a variation of the donor chamber with a piston biasing means; and
FIGURE 9 shows a variation of the donor chamber with sealing caps or plugs.

### DETAILED DESCRIPTION -

### i) Donor Chamber (Figures 1, 2, 3, 4, 7, 8, 9)

The donor chamber 1 is composed of clear plastic, and consists of a curved chamber, open at both ends. The donor lenticule 2 is dragged into the chamber and coiled up in a specific proprietary configuration, with the stromal surface snugly aligned to the inner surface of the chamber 1. The front end of the chamber 1 is bevelled in a cut-away design to allow ease of insertion through a scleral wound opening 3. The outer chamber diameter is designed to fit exactly through the scleral wound 3 with a semi-elliptical opening. The superior aspect of the anterior edge has a cut-away anterior surface to allow space for a pair of glide forceps (not shown) to enter the chamber 1 and grasp the leading stromal edge of the coiled donor 2. The posterior margins of the chamber 1 are straight-edged and grooved, to allow sliding of a glide platform 4 to close the chamber 1 inferiorly, with a glide member 5 protruding anteriorly (this will be inserted into the AC). The posterior back surface is also open, but will be closed by the posterior, matching back which is part of the glide platform 4.

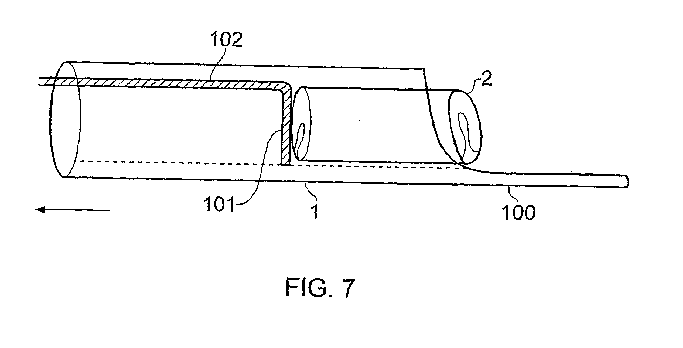

With particular reference to Figure 7, there is shown a donor chamber 1 with a bevelled cut-away insertion end 100 and a coiled donor 2 located in the chamber 1. A biasing means in the form of a plate 101 on the end of a push-rod 102 is provided, the biasing means being similar in configuration to a croupier's rake. The biasing means can be used to push the donor 2 out of the chamber 1 into the recipient's eye when the insertion end 100 has been inserted through the scleral incision 3.

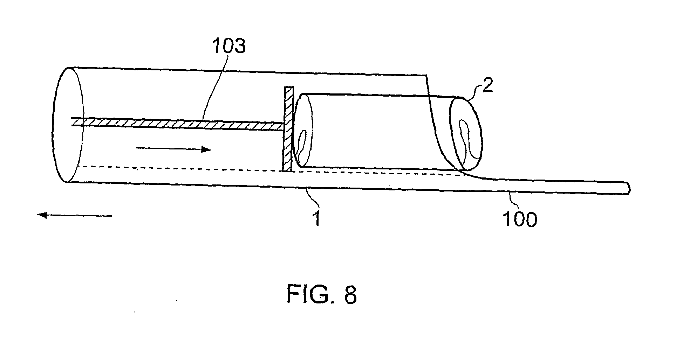

Figure 8 shows a further variation in which the biasing means is configured as a piston 103.

In both the Figure 7 and the Figure 8 embodiments, the biasing means may be advanced while the chamber 1 is held stationary, or the biasing means may be held stationary while the chamber 1 is retracted..

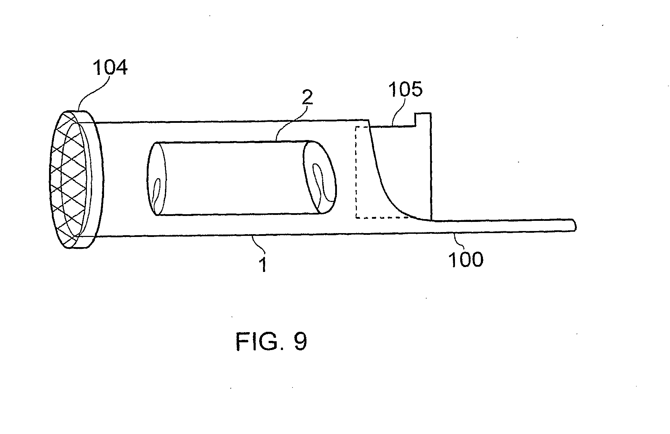

Figure 9 shows a variation in which the donor chamber 1 is provided with a plug or seal 104, 105 at each end so as to allow the donor 2 to be stored in the chamber 1 in a suitable nutrient solution. In this way, the donor chamber 1 can be pre-filled with an appropriate donor 2 at an eye bank or the like, before being shipped to a surgeon in the form of a ready-to-use cartridge. This is particularly useful when the donor 2 requires special preparation after excision from a cadaver, since employees at the eye bank may be more skilled at such preparation than a surgeon, who will generally be more skilled at inserting the donor 2 into a recipient eye.

### ii) Glide Platform (Figures 1, 2, 4)

The glide platform 4 is composed of clear plastic, and has three sections. The anterior section 5 forms a flat glide portion which will be inserted into the AC. This is approximately 0.3mm thick and slightly flexible. Mid-way along the glide surface, there is provided a 1.0 cm long central vertical protruding ridge 6 with curved margins present on the anterior aspect of the glide. The vertical ridge 6, which is 0.5mm in height, enables the widest margins of the coiled donor 2 to coil up internally, stromal surface touching, hence preventing inadvertent endothelial surface touch which may occur if one edge of the donor 2 slides over the other. The middle portion 7 consists of a continuation of the anterior glide, which will form the base platform of the donor chamber 1, and the raised posterior back 8 of the chamber 1 (a back cowling) which attaches to the posterior aspect of the chamber 1, creating a closed chamber posteriorly. The posterior portion 9 consists of a broad base platform (approximately 2.5 cm thick) which may be flat or curved (concave surface upwards) and which will be the portion of the platform 4 which is grasped by the surgeon's thumb and forefinger during insertion. Ridges may be placed to avoid slippage. Threading the donor chamber 1 onto the glide platform 4 forms a complete thumb drive device, with a chamber holding the donor which will be completely coiled within.

A further design modification of the donor chamber 1 takes into account that the anterior opening of the donor chamber 1 will need to be inserted into the scleral wound 3 and introduction into the wound 3 with this large diameter bore may result in excessive outflow of chamber fluid and lead to temporary shallowing during introduction of the glide. The alternative design modification provides a thin, flexible, anterior cover flap over the donor chamber 1 opening, hinged superiorly. This will be an integral anterior extension of the upper surface of the chamber opening, and the flap may be inserted depressed down and tucked beneath the upper lip of the scleral wound 3, thus providing a temporary seal to the anterior opening of the donor chamber 1, thus reducing aqueous outflow and shallowing of the AC. Once the anterior margins of the donor chamber 1 have fully entered the scleral wound 3 and protrude into the AC, the flexible roof of this cover will spring upwards back into its original configuration, reopening the donor chamber 1 bore, thus allowing for the donor cornea 2 to be pulled unimpeded into the AC.

### iii) Glide Forceps

Where provided, this consists of a curved, single shaft intraocular titanium forceps designed to enter a 1 mm paracentesis passed through a nasal corneal opening opposite the temporal scleral incision 3. The forceps tip consists of a sharp-tipped opening and closing forceps design similar to other designs for intraocular capsulorhexis forceps but with a vertical orientation of the opening and closing teeth, as opposed to horizontal orientation in conventional capsulorhexis forceps. The forceps will be used to grasp the leading stromal edge of the coiled donor 2, while within the glide chamber, and used to pull the donor 2 through the donor chamber 1 into the AC.

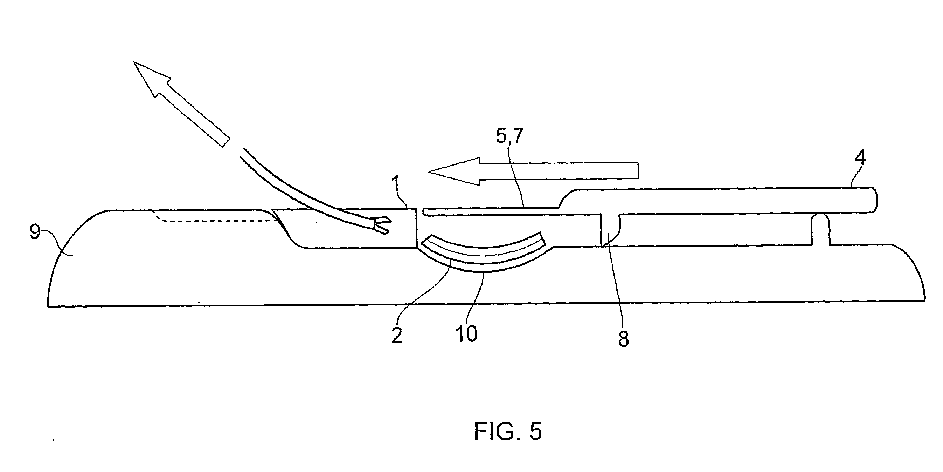

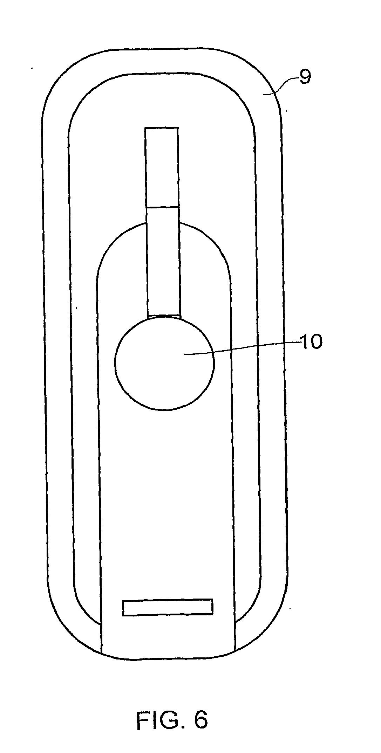

### iv) Preparation Base (Figures 5, 6)

The preparation base 9 consists of plastic (or may be made of any other suitable material), and enables placement of the corneal donor 2 in position prior to coiled insertion into the donor chamber 1. The base 9 also enables stable positioning of the donor chamber 1 while the donor 2 is pulled into the chamber 1, and enables sliding closure of the glide platform 4 into the locked position on the donor chamber 1.

### Thumb Glide donor insertion technique

Stages of donor coiling, thumb glide assembly, insertion of the thumb glide through the scleral wound, donor pull-through, and removal of the thumb glide are described:
1. The donor chamber 1 is placed onto the preparation base 9, upside down.
2. The donor cornea 2 (consisting of both the posterior lenticule and attached accompanying anterior cap) is placed, endothelial surface up, onto the circular platform 10 of the base 9. The donor 2 will have already been lamellar dissected and trephined to the desired diameter prior to placement, and liberally coated with dispersive OVD over the endothelial surface. One drop of BSS is placed on the circular platform 10 prior to donor placement to lubricate the surface and prevent the donor 2 sticking to the surface of the circular platform 10. Care should be taken to ensure that OVD is not present between the posterior donor stromal surface and does not overflow over the donor peripheral edges.
3. Careful loosening of the posterior donor lenticule from the anterior stromal cap is performed by gently separating the contacting surfaces with a BSS cannula, with injection of a small amount of BSS to lubricate the adjoining surfaces. This will allow easier separation of the posterior lenticule from the anterior cap.
4. The anterior leading stromal edge of the posterior lenticule is carefully marked with a surgical gentian violet marker pen so as to aid visualization of leading stromal edge.
5. The glide forceps is then used to grasp the inked leading stromal edge of the posterior lenticule, and is next used to carefully pull the donor 2 into the donor chamber 1, leaving the anterior cap behind, ensuring that there is full adherence of the stromal surface of the posterior donor to the inner margins of the donor chamber 1 (Figure 5). One drop of BSS on the inner surface of the chamber 1 may reduce friction during this process. The curved surface of the chamber 1 will initiate concave coiling of the donor 2. If detachment or wrinkling of the donor 2 occurs during the initial pulling process, a second forceps can be use to guide the edges of the donor 2 to ensure that the donor coils accurately without wrinkling, is centred, and in direct contact with the inner edges of the chamber 1 at all times. The donor 2 is pulled all the way in into the chamber 1, until the leading edge of the donor 2 is approximately 0.5mm from the anterior margin of the chamber 1. At this stage, the central outer edges of the donor lenticule will protrude vertically above the chamber sides.
6. The glide platform 4 is now be used to close the chamber 1. The leading glide edge of the glide platform 4 (which is introduced upside down) is threaded through the grooves of the chamber margins from the posterior margin and advanced slowly (Figure 5). As the anterior flat portion of the glide encounters the vertically protruding central edges of the donor lenticule, careful further advancement will fold the donor edges inwards. Forceps may be used to tuck in the edges if necessary. When the edges then encounter the vertical protruding groove of the glide mid-way along insertion, the edges will be further coiled downwards, in a double coil configuration - the stromal surfaces will be in contact with each other and fold inwards more (this ensures that endothelial surfaces do not slide over each other). The glide will then be fully advanced until the posterior cap of the glide encounters the posterior margins of the donor chamber 1 and the glide is clipped into position over the chamber 1, ensuring a tight posterior seal. The complete thumb glide is now fully assembled, and can be fully detached from the preparation base 9, re-inverted upright, and set aside for insertion.
7. Recipient preparation: At this stage, the recipient cornea should already have been prepared (surface epithelium should already have been removed (if needed), trephination marking on the corneal surface performed, the scleral wound fashioned, nasal paracentesis performed, AC maintainer placed (either at superior or inferior limbus), and a peripheral iridectomy performed).
8. Descemet's stripping (if needed) is performed in the usual manner.
9. Introduction of the thumb glide containing the donor: With the surgeon holding the thumb glide with the thumb and forefinger (right hand for a right-handed surgeon), the anterior glide portion of the thumb glide is first inserted through the wound, with the AC maintainer on. The glide is advanced until the anterior edge of the donor chamber engages the superior lip of the scleral wound. The donor chamber edge is carefully slid beneath the superior lip, using forceps to lift the wound edge if necessary. The full process of insertion should be quick and deliberate, so as not to lose BSS and cause excessive chamber shallowing. The glide is then advanced fully through the wound 3, until the superior edge of the donor chamber 1 is just beyond the limbus, at which point the inked edge of the donor will be just visible through the peripheral clear cornea. At this stage, the glide will be fully engaged at the wound 3, which will be stretched open by the chamber diameter. Elasticity of the sclera will enable the glide to be fully pressed against the wound opening, ensuring a tight seal with minimal leakage of BSS. The AC should be fully maintained and deep throughout this procedure, with the AC maintainer on, and minimal egress of BSS will ensure that the donor remains stable within the donor chamber 1 at this point.
10. The glide forceps is introduced through the nasal paracentesis opening, with the surgeon's left hand, and advanced across the AC above the glide. The forceps tip is passed just beneath and beyond the donor chamber 1 and the teeth opened to grasp the leading stromal edge of the donor 2 which can be clearly visualized by the ink marking. The forceps is then gently retracted out of the AC, pulling the donor 2 smoothly into the AC. As the donor 2 enters the AC, it will unfold by itself in the deep chamber. If incomplete folding of the outer edges occurs, the forceps may be used to gently agitate the donor sideways back and forth, to facilitate full unfolding. The forceps can also be used to position the donor 2 centrally in alignment with the superficial trephination on the recipient corneal surface. At this point, the grip on the donor is not yet released.
11. Once the donor 2 is fully unfolded, and in position, the donor glide should be smoothly but quickly retracted completely out of the wound, allowing the scleral wound 3 to close. Some egress of BSS will be encountered during this manoeuvre, but BSS inflow from the AC maintainer will rapidly refill and deepen the AC. Continual holding onto the donor with the forceps will ensure that the donor does not slip out of the wound. If the slightly stretched scleral wound remains partially open, an assistant may help to close the scleral wound 3. Once the glide is fully retracted and the AC fully reformed, a small air bubble may be injected into the AC, beneath the donor, by the right hand of the surgeon with a 30 gauge needle through a separate limbal site - this will float the donor up against the posterior corneal surface, ensuring that the donor does not settle downwards onto the iris or IOL surface. Finally, the donor 2 may now be fully released from the forceps. Completion of the DSAEK procedure by suturing the wound and paracentesis sites, followed by a full air injection for donor adherence, can now be performed and the procedure completed in the usual manner.

Alternative Thumb Glide insertion techniques are also currently being explored. In particular one alternative is to modify the glide to enable near-complete insertion of the entire glide (containing the donor) into the AC, thereby positioning the coiled cornea safely in a controlled manner within the centre of the AC. The cornea is then much closer to the opposite chamber angle, enabling a shorter glide forceps to be used. The glide is then simply retracted out of the AC, leaving the donor in position to uncoil of its own accord.

### Novel aspects of preferred embodiments of the present invention:

There are several novel and unique concepts of embodiments and techniques of and involving the present invention, which are listed below. The list is not exhaustive, and the advantages may be obtained individually or in any appropriate combination.
1. Glide concept - coupled with the use of an AC maintainer on throughout the procedure, a deep chamber is maintained at all times, the glide prevents iris prolapse out of the wound and prevents contact of the endothelial surface of the donor with iris or IOL surfaces.
2. Close chamber concept of donor insertion - the closed chamber of the thumb glide ensures no turbulent outflow of BSS during insertion, and a stable, deep and quiet chamber. The chamber of the glide acts as an extension of the AC, allowing for safe and simple donor entry into the AC.
3. The clear plastic design of the glide allows for full visualization of the donor and AC details at all time, facilitating accurate grasping of the donor.
4. The disposable nature of the glide ensures no risk of prion disease and other microbial and biofilm contamination inherent in non-disposable alternatives which need cleaning and sterilization.
5. The unique double coiling principle of the donor within the glide chamber allows for minimal endothelial contact with stroma or adjacent endothelial surfaces, thus reducing endothelial damage. The circular coiling of the donor also avoids sharp folding creases of the donor, again limiting endothelial cell damage. The double coiling shape also minimizes that total diameter of the glide, thus enabling the smallest wound size to be achieved with no donor touch, coupled with an ideal elliptical profile of the internal diameter of the glide. Various different glide sizes (three, for example) enable the surgeon to select the appropriate glide size according to the desired wound size, donor diameter, and thickness of the donor lenticule.
6. The donor is completely protected from contact with the wound edges during insertion, by the fact that the walls of the glide abut the entire wound margin, thus further reducing endothelial damage during insertion.

## Claims

1. An assembly for resiliently deforming a corneal implant (2), the assembly comprising a deformation chamber comprising:
a housing member (1) having an internal curved surface, said housing member (1) arranged to receive and hold said implant (2) in a curved orientation, such that peripheral edges of said implant (2) are directed away from said housing member;
a platform (7,9) in sliding engagement with said housing member (1), said platform (7,9) having a profiled face arranged to contact said implant (2) on sliding said housing member (1) into contact with said profiled face; wherein
said profiled face is shaped to direct said peripheral edges towards said housing member (1) on contact through said sliding engagement.

2. An assembly as claimed in claim 1, wherein the curved orientation is any one of: a double coil, a cardioid, reverse curve, reverse double curve or corrugated.

3. An assembly as claimed in claim 1 or 2, wherein the profiled face includes a ridge (6) that projects into the housing member (1), the ridge (6) preferably extending in a direction substantially parallel to a longitudinal axis of the platform (7,9).

4. An assembly as claimed in claim 1 or 2, wherein the platform (7,9), when engaged with the housing member (1), has a portion (7) forming a lid for the housing member (1), and wherein the portion includes a ridge (6) that projects into the housing member (1).

5. An assembly as claimed in any preceding claim, wherein the housing member (1) is a deformation chamber for storing said implant (2) in a pre-determined deformed shape, said platform (7) having an insertion end (5) for inserting into a corneal incision; further comprising biasing means for biasing the implant (2) from the housing (1) whilst in the deformed shape.

6. An assembly as claimed in claim 5, wherein the biasing means comprises forceps arranged to engage and pull the implant (2) into place.

7. An assembly as claimed in claim 5, wherein the biasing means comprises an element (101,102,103) for pushing the implant (2) into place.

8. An assembly as claimed in claim 5, wherein the biasing means is configured to engage a surface of the implant (2), through friction adhesion or other such means, and to draw the implant (2) from the assembly into place.

9. An assembly as claimed in any preceding claim, further comprising a preparation base (9) having a concave well (10) for receiving the implant (2), means on one side of the well (10) for receiving the housing (1), and means on an opposed side of the well (10) for guiding the platform (7,9) into engagement with the housing (1).

10. An assembly as claimed in claim 9, further comprising means for moving the implant (2) from the well (10) into the housing (1) prior to or during engagement of the platform (7,9) with the housing (1).

11. An assembly as claimed in any preceding claim, wherein the housing member (1) is provided with removable sealing members (104,105) to allow the implant (2) to be stored in the housing member (1) for transportation.

12. A method of preparing a corneal implant (2) for insertion into a recipient patient, the method comprising the steps of:
providing i) a preparation base (9) having a concave well (10) for receiving the implant (2); ii) a housing (1); and iii) a platform (7,9) having a portion adapted slidably to engage with and form a cover for the housing (1);
locating the housing (1) in retaining means provided on the preparation base (9) on one side of the concave well (10);
locating the platform (7,9) on guiding means provided on the preparation base (9) on an opposed side of the concave well (10);
providing a corneal implant (2) and locating this in the concave well (2);
and moving the implant (2) into the housing (1) from the concave well (10), prior to or at the same time as moving the platform (7,9) on the guiding means such that the portion of the platform (7,9) engages with the housing (1) and contains the implant (2) therein.

13. A method according to claim 12, wherein the implant (2) is moved by way of forceps.

14. A method according to claim 12 or 13, wherein the housing (1) and/or the portion of the platform (7,9) is shaped so as to cause the implant (2) to assume a curved configuration in the housing (1 ) when contained therein.

15. A method according to claim 14, wherein the curved orientation is any one of: a double coil, a cardioid, reverse curve, reverse double curve or corrugated.

## Patentansprüche

1. Einheit zum elastischen Verformen eines Hornhautimplantats (2), wobei die Einheit eine Verformungskammer umfasst, die folgendes umfasst:
ein Gehäuseelement (1) mit einer inneren gekrümmten Oberfläche, wobei das Gehäuseelement (1) so angeordnet ist, dass es das Implantat (2) in einer gekrümmten Ausrichtung aufnimmt und hält, so dass periphere Kanten des Implantats (2) von dem Gehäuseelement weggehend gerichtet sind;
eine Plattform (7, 9), die sich in gleitfähigem Eingriff mit dem Gehäuseelement (1) befindet, wobei die Plattform (7, 9) eine profilierte Fläche aufweist, die so angeordnet ist, dass sie das Implantat (2) berührt, wenn das Gehäuseelement (1) in Kontakt mit der profilierten Fläche geschoben wird; wobei
die profilierte Fläche so geformt ist, dass sie die peripheren Kanten bei Kontakt über den gleitfähigen Eingriff in Richtung des Gehäuseelements (1) richtet.

2. Einheit nach Anspruch 1, wobei die gekrümmte Ausrichtung eine der folgenden ist: eine Doppelspule, eine Herzkurve, eine S-Kurve, eine S-Doppelkurve oder eine gewellte Ausrichtung.

3. Einheit nach Anspruch 1 oder 2, wobei die profilierte Fläche eine Rippe (6) aufweist, die in das Gehäuseelement (1) vorsteht, wobei sich die Rippe (6) vorzugsweise in eine Richtung erstreckt, die im Wesentlichen parallel ist zu einer Längsachse der Plattform (7, 9).

4. Einheit nach Anspruch 1 oder 2, wobei die Plattform (7, 9), wenn sie mit dem Gehäuseelement (1) eingreift, einen Abschnitt (7) aufweist, der einen Deckel für das Gehäuseelement (1) bildet, und wobei der Abschnitt eine Rippe (6) aufweist, die in das Gehäuseelement (1) vorsteht.

5. Einheit nach einem der vorstehenden Ansprüche, wobei das Gehäuseelement (1) eine Verformungskammer zur Aufbewahrung des Implantats (2) in einer vorbestimmten verformten Form ist, wobei die Plattform (7) ein Einführungsende (5) zum Einführen in einen Hornhauteinschnitt aufweist; ferner umfassend eine Vorbelastungseinrichtung zur Vorbelastung des Implantats (2) von dem Gehäuse (1), während es sich in der verformten Form befindet.

6. Einheit nach Anspruch 5, wobei die Vorbelastungseinrichtung eine Pinzette umfasst, die so angeordnet ist, dass sie mit dem Implantat (2) eingreifen und dieses an eine Verwendungsposition ziehen kann.

7. Einheit nach Anspruch 5, wobei die Vorbelastungseinrichtung ein Element (101, 102, 103) umfasst, das dazu dient, das Implantat (2) an eine Verwendungsposition zu drücken.

8. Einheit nach Anspruch 5, wobei die Vorbelastungseinrichtung so konfiguriert ist, dass sie mit einer Oberfläche des Implantats (2) durch Reibschluss oder andere derartige Mittel eingreift und das Implantat (2) aus der Einheit an eine Verwendungsposition ziehen kann.

9. Einheit nach einem der vorstehenden Ansprüche, wobei diese ferner eine Präparationsbasis (9) mit einer konkaven Senke (10) zur Aufnahme des Implantats (2), mit Mitteln auf einer Seite der Senke (10) zur Aufnahme des Gehäuses (1) und mit Mitteln auf einer entgegengesetzten Seite der Senke (10) zum Führen der Plattform (7, 9) in Eingriff mit dem Gehäuse (1) umfasst.

10. Einheit nach Anspruch 9, wobei diese ferner Mittel zum Bewegen des Implantats (2) von der Senke (10) in das Gehäuse (1) umfasst, vor oder während dem Eingriff der Plattform (7, 9) mit dem Gehäuse (1).

11. Einheit nach einem der vorstehenden Ansprüche, wobei das Gehäuseelement (1) mit entfernbaren Verschlusselementen (104, 105) versehen ist, um eine Aufbewahrung des Implantats (2) in dem Gehäuseelement (1) für Transportzwecke zu ermöglichen.

12. Verfahren zur Vorbereitung eines Hornhautimplantats (2) auf das Einsetzen in einem empfangenden Patienten, wobei das Verfahren die folgenden Schritte umfasst:
Bereitstellen i) einer Präparationsbasis (9) mit einer konkaven Senke (10) zur Aufnahme des Implantats (2); ii) eines Gehäuses (1); und iii) einer Plattform (7, 9) mit einem Abschnitt, der gleitfähig mit dem Gehäuse (1) eingreifen kann und eine Abdeckung für dieses bilden kann;
Positionieren des Gehäuses (1) in Haltemitteln, die an der Präparationsbasis (9) auf einer Seite der konkaven Senke (10) angeordnet sind;
Positionieren der Plattform (7, 9) an Führungsmitteln, die an der Präparationsbasis (9) auf einer zu der konkaven Senke (10) entgegengesetzten Seite angeordnet sind;
Bereitstellen eines Hornhautimplantats (2) und Positionieren des Implantats in der konkaven Senke (2);
und Bewegen des Implantats (2) von der konkaven Senke (10) in das Gehäuse (1), vor oder gleichzeitig zu dem Bewegen der Plattform (7, 9) an den Führungsmitteln, so dass der Abschnitt der Plattform (7, 9) mit dem Gehäuse (1) eingreift und das Implantat (2) darin aufweist.

13. Verfahren nach Anspruch 12, wobei das Implantat (2) durch eine Pinzette bewegt wird.

14. Verfahren nach Anspruch 12 oder 13, wobei das Gehäuse (1) und/oder der Abschnitt der Plattform (7, 9) so geformt ist, dass bewirkt wird, dass das Implantat (2) eine gekrümmte Konfiguration in dem Gehäuse (1) annimmt, wenn es sich darin befindet.

15. Verfahren nach Anspruch 14, wobei die gekrümmte Ausrichtung eine der folgenden ist: eine Doppelspule, eine Herzkurve, eine S-Kurve, eine S-Doppelkurve oder eine gewellte Ausrichtung.

## Revendications

1. Ensemble destiné à déformer élastiquement un implant cornéen (2), l'ensemble comprenant une chambre de déformation, comprenant :
un élément boîtier (1) ayant une surface courbe interne, ledit élément boîtier (1) étant conçu pour recevoir et maintenir ledit implant (2) dans une orientation courbée, de sorte que les bords périphériques dudit implant (2) sont dirigés à l'opposé dudit élément boîtier ;
une plate-forme (7, 9) en prise coulissante avec ledit élément boîtier (1), ladite plate-forme (7, 9) ayant une face profilée conçue pour entrer en contact avec ledit implant (2) en faisant coulisser ledit élément boîtier (1) en contact avec ladite face profilée ; dans lequel
ladite face profilée est façonnée pour diriger lesdits bords périphériques vers ledit élément boîtier (1) en contact à travers ladite prise coulissante.

2. Ensemble selon la revendication 1, dans lequel l'orientation courbe est l'une quelconque de : une bobine double, une cardioïde, une courbe inversée, une courbe double inversée ou ondulée.

3. Ensemble selon la revendication 1 ou 2, dans lequel la face profilée comprend une nervure (6) qui fait saillie dans l'élément boîtier (1), la nervure (6) s'étendant de préférence dans une direction sensiblement parallèle à un axe longitudinal de la plate-forme (7, 9).

4. Ensemble selon la revendication 1 ou 2, dans lequel la plate-forme (7, 9), lorsqu'elle est prise avec l'élément boîtier (1), a une partie (7) formant un couvercle pour l'élément boîtier (1), et dans lequel la partie comprend une nervure (6) qui fait saillie dans l'élément boîtier (1).

5. Ensemble selon l'une quelconque des revendications précédentes, dans lequel l'élément boîtier (1) est une chambre de déformation pour stocker ledit implant (2) dans une forme déformée prédéterminée, ladite plate-forme (7) ayant une extrémité d'insertion (5) destinée à être insérée dans une incision de la cornée ; comprenant en outre des moyens de sollicitation pour solliciter l'implant (2) à partir du boîtier (1) en étant en forme déformée.

6. Ensemble selon la revendication 5, dans lequel le moyen de sollicitation comprend une pince conçue pour venir en prise avec et tirer l'implant (2) en place.

7. Ensemble selon la revendication 5, dans lequel le moyen de sollicitation comprend un élément (101, 102, 103) pour pousser l'implant (2) en place.

8. Ensemble selon la revendication 5, dans lequel le moyen de sollicitation est conçu pour venir en prise avec une surface de l'implant (2), à travers une adhérence par frottement ou d'autres moyens, et pour tirer l'implant (2) à partir de l'ensemble en place.

9. Ensemble selon l'une quelconque des revendications précédentes, comprenant en outre une base de préparation (9) ayant un puits concave (10) destiné à recevoir l'implant (2), un moyen sur un côté du puits (10) destiné à recevoir le boîtier (1), et un moyen sur un côté opposé du puits (10) destiné à guider la plate-forme (7, 9) en prise avec le boîtier (1).

10. Ensemble selon la revendication 9, comprenant en outre un moyen pour déplacer l'implant (2) du puits (10) dans le boîtier (1), avant ou pendant la mise en prise de la plate-forme (7, 9) avec le boîtier (1).

11. Ensemble selon l'une quelconque des revendications précédentes, dans lequel l'élément boîtier (1) est pourvu d'éléments d'étanchéité amovibles (104, 105) pour permettre à l'implant (2) d'être stocké dans l'élément boîtier (1) à des fins de transport.

12. Procédé de préparation d'un implant cornéen (2) destiné à être inséré dans un patient receveur, le procédé comprenant les étapes consistant à :
fournir i) une base de préparation (9) ayant un puits concave (10) destiné à recevoir l'implant (2) ; ii) un boîtier (1) ; et iii) une plate-forme (7, 9) ayant une partie conçue pour venir en prise de façon coulissante avec et former un couvercle pour le boîtier (1) ;
placer le boîtier (1) dans un moyen de retenue prévu sur la base de préparation (9) sur un côté du puits concave (10) ;
placer la plate-forme (7, 9) sur le moyen de guidage prévu sur la base de préparation (9) sur un côté opposé du puits concave (10) ;
fournir un implant cornéen (2) et le placer dans le puits concave (2) ;
et déplacer l'implant (2) dans le boîtier (1) à partir du puits concave (10), avant ou en même temps que le déplacement de la plate-forme (7, 9) sur le moyen de guidage de sorte que la partie de la plate-forme (7, 9) vient en prise avec le boîtier (1) et contient l'implant (2) à l'intérieur.

13. Procédé selon la revendication 12, dans lequel l'implant (2) est déplacé au moyen d'une pince.

14. Procédé selon la revendication 12 ou 13, dans lequel le boîtier (1) et/ou la partie de la plate-forme (7, 9) est conformé de manière à amener l'implant (2) à prendre une configuration courbe dans le boîtier (1) lorsqu'il est placé à l'intérieur de celui-ci.

15. Procédé selon la revendication 14, dans lequel l'orientation courbe est l'une quelconque de : une bobine double, une cardioïde, une courbe inversée, une courbe double inversée ou ondulée.
